# EUROPÄISCHE PATENTSCHRIFT

(11) **EP 2 182 966 B1**
(45) Veröffentlichungstag und Bekanntmachung des Hinweises auf die Patenterteilung: **08.08.2012**
(21) Anmeldenummer: 08783450.3
(22) Anmeldetag: 15.08.2008
(51) Int. Cl.: A61K 36/67, A61P 35/00

(54) **VERWENDUNG VON EXTRAKTEN ODER EXTRAKTIVSTOFFEN AUS PIPER CUBEBA L. ALS WIRKSAME BESTANDTEILE IN EINEM MEDIKAMENT ZUR BEHANDLUNG VON KREBSERKRANKUNGEN**
THE USE OF EXTRACTS OR MATERIALS EXTRACTED FROM PIPER CUBEBA L. AS AN EFFECTIVE COMPONENT IN A DRUG FOR THE TREATMENT OF CANCER DISEASES
UTILISATION D'EXTRAITS OU DE SUBSTANCES EXTRACTIVES DE PIPER CUBEBA L. COMME PRINCIPES ACTIFS DANS UN MÉDICAMENT DESTINÉ AU TRAITEMENT DE PATHOLOGIES CANCÉREUSES

(30) Priorität: 16.08.2007 CH 12892007
(43) Veröffentlichungstag der Anmeldung: 12.05.2010
(73) Patentinhaber: Viridis Pharmaceutical Limited, Road Town Tortola (VG)
(72) Erfinder: KREUTER, Matthias-Heinrich, CH-8880 Walenstadt (CH); YAM, Jian, Ying, CH-8808 Pfäffikon SZ (CH); BERGER-BÜTER, Karin, CH-4108 Witterswil (CH)
(74) Vertreter: Hasler, Erich
(86) Internationale Anmeldenummer: PCT/CH2008/000350
(87) Internationale Veröffentlichungsnummer: WO 2009/021347

(56) Entgegenhaltungen:
- CHATTERJEE A ET AL: "SPECTRAL PROPERTIES OF CUBEBIN PIPER-CUBEBA-D" JOURNAL OF THE INDIAN CHEMICAL SOCIETY, THE INDIAN CHEMICAL SOCIETY, CALCUTTA, IN, Bd. 45, Nr. 8, 1. Januar 1968 (1968-01-01), Seiten 723-725, XP009092178 ISSN: 0019-4522
- DASGUPTA A ET AL: "MEDICINAL SPECIES OF PIPER, PHARMACOGNOSTIC DELIMITATION" QUARTERLY JOURNAL OF CRUDE DRUG RESEARCH, SWETS AND ZEITLINGER, LISSE, NL, Bd. 18, Nr. 1, 1. Januar 1980 (1980-01-01), Seiten 17-25, XP009006829 ISSN: 0033-5525
- CHOI EUN-MI ET AL: "Investigations of anti-inflammatory and antinociceptive activities of Piper cubeba, Physalis angulata and Rosa hybrida" JOURNAL OF ETHNOPHARMACOLOGY, ELSEVIER SCIENTIFIC PUBLISHERS LTD, IE, Bd. 89, Nr. 1, 1. November 2003 (2003-11-01), Seiten 171-175, XP009092358 ISSN: 0378-8741

## Beschreibung

Die vorliegende Erfindung betrifft die Verwendung von Extrakten oder Extraktivstoffen aus Piper cubeba L. als wirksame Bestandteile in einem Medikament zur Behandlung von Krebserkrankungen.

Verwendungen von Piper cubeba L. Zubereitungen sind beispielsweise beschrieben in Hunnius, Pharmazeutisches Wörterbuch, 8. Auflage, 1998, Seiten 1084 bis 1085.

Hierin werden volkstümliche Anwendungen, wie die Behandlung von Kopfschmerzen sowie die Verwendung als Diuretikum, Harndesinfizienz und Stomachikum beschrieben.

Als Droge wird die unreife Frucht verwendet, aus der auch Kubeben-Öl, das ätherische Öl der Kubeben, durch Wasserdampfdestillation gewonnen wird.

Oleum Cubebae wird in den gleichen Indikationen wie die Früchte eingesetzt.

Gemäss J. Seidemann in "World Spice Plants", Springer-Verlag, 2005, Seite 291 wird Piper cubeba L. zur Aromatisierung von Likören, Ingwerbrot und Honigbrot verwendet. Als Produkt dient das ätherische Öl, das aus der unreifen Frucht gewonnen wird.

In JP 2000-095649 A werden Extrakte beschrieben, unter anderem auch aus der Kubeben-Frucht, die mittels eines hydrophilen Lösungsmittels, beispielsweise Aceton, Methanol und Ethanol, oder deren Mischungen mit Wasser erhalten werden. Solche Extrakte enthalten somit sowohl das ätherische Öl als auch hydrophile Substanzen.

Diese Extrakte sollen als Testosteron-5α-Reduktasehemmer wirken. Dadurch sollen diese Extrakte den Haarwuchs positiv beeinflussen.

Diese Extrakte sollen auch zur Behandlung von benigner Prostatahyperplasie dienen.

In diesem Dokument werden in Tabelle 1 IC₅₀-Werte aufgeführt, die sich auf die Hemmung der Testosteron-5α-Reduktase beziehen.

Der Extrakt aus Kubeben hemmt laut dieser Tabelle das Enzym zu 50% bei einer Konzentration von 0,79 mg/ml.

A. Chatterjee et .al., Jour. Indian Chem. Soc., Vol. 45, No. 8, 1968, Seiten 723 bis 725 beschreibt die spektralen Eigenschaften der Reinsubstanz Cubebin. Diese chemische Verbindung wurde aus einem alkoholischen Extrakt der entfetteten Früchte von Piper cubeba L. isoliert. Die Anwendung von Cubebin wird in der Heilung von Schleimhäuten bei chronischen Krankheiten gesehen.

In der WO 03/080600 ist ein Verfahren zur Gewinnung von Lignanen, darunter die Reinsubstanz Cubebin, aus Blättern von Zanthoxylum naranjillo oder aus Piper cubeba beschrieben. Dazu werden die Blätter erst getrocknet und gemahlen. Das entstehende Pulver wird mittels Hexan extrahiert und das Extrakt wird filtriert sowie konzentriert. Eine wiederholte Reinigung mittels Chromatographie und Kristallisation führt zum Reinstoff. Es wird erwähnt, dass die Klasse der Lignane anti-Tumor und anti-virale Aktivitäten zeigen.

A. Dasgupta et.al., Quart. J. Crude Drug Res., 18, (1980), No. 1, Seiten 17 bis 25 beschreibt die Verwendung des ätherischen Öles von Piper cubeba L. zur Behandlung von beispielsweise Cystitis, und Gonorrhoe.

Eun-Mi Choi et. al., Journal of Ethnopharmacology, Elsevier Scientific Publishers Ltd., 89, 2003, Seiten 171 bis 175 beschreibt entzündungshemmende Eigenschaften eines mit 80%-igem Methanol hergestellten Extraktes aus den getrockneten Früchten von Piper cubeba L..

Im Rahmen eines Screening-Verfahrens wurde eine Reihe tropischer Arzneipflanzen auf ihre Wirkung gegenüber Tumorzellen *in vitro* untersucht.

Völlig überraschend wurde gefunden, dass ein ethanolischer Extrakt aus unreifen Früchten von Kubeben alle getesteten Tumorzellen abtötete.

Da in erster Linie das ätherische Öl der Kubeben-Früchte medizinisch verwendet wird, war es naheliegend, die Früchte mit einem geeigneten Extraktionsmittel zu extrahieren, um das ätherische Öl zu erhalten.

Dies wurde durch erschöpfende Extraktion mit Hexan realisiert.

Die so vom ätherischen Öl befreiten Früchte wurden der Vollständigkeit halber zusätzlich mit 90%-igem wässrigem Ethanol extrahiert, um mittelpolare Extraktivstoffe zu erhalten.

Sowohl das gewonnene ätherische Öl als auch der ethanolische Sekundärextrakt wurden anschliessend auf ihre Aktivitäten gegenüber Tumorzellen geprüft.

Es wurde wie erwartet festgestellt, dass das gewonnene ätherische Öl alle getesteten Tumorzellen abtötete. Dies deutet darauf hin, dass der beobachtete zytotoxische Effekt unspezifischer Natur ist und damit keinen Antitumor-Effekt darstellt.

Völlig überraschend wurde aber gefunden, dass der ethanolische Sekundärextrakt zwar keine der getesteten Tumorzellen direkt abtötete, aber bei einigen Tumorzellen deren Proliferationsverhalten veränderte.

Solche Tumorzellen erwiesen sich als besonders empfindlich gegenüber dem ethanolischen Sekundärextrakt, die für ihr Wachstum Sexualhormone als Wachstumsfaktoren benötigen. Als Beispiele seien die Brustkrebs-Zelllinie MCF 7 und die Prostatakrebs-Zelllinie LnCAP genannt. Diese Beobachtung lässt den Schluss zu, dass die proliferationshemmende Wirkung nicht primär auf einer Hemmung der Testosteron-5α-Reduktase beruhen kann, da diese für das Wachstum der Brustkrebs-Zelllinie MCF 7 unerheblich ist.

Es ist ein Ziel der vorliegenden Erfindung, ein Verfahren zur Herstellung eines Extraktes aus Früchten von Piper cubeba L. zur Verfügung zu stellen.

Dieser Extrakt soll frei oder nahezu frei von zytotoxischen ätherischen Ölen sein.

Dieser Extrakt soll das Wachstum insbesondere von solchen Tumorzellen hemmen, die für ihr Wachstum Sexualhormone als Wachstumsfaktoren benötigen.

Dieser Extrakt soll anti-androgene und/oder anti-östrogene Wirkungen aufweisen.

Dieser Extrakt soll die Wirkungen des Sexualhormons Dihydrotestosteron, abgekürzt mit DHT, antagonisieren, insbesondere dessen proliferationssteigernde und anti-apoptotische Wirkung auf Prostatakrebszellen.

Diese Ziele werden mit der vorliegenden Erfindung erreicht.

Die Erfindung ist durch die Merkmale in den unabhängigen Ansprüchen gekennzeichnet.

Dazu gehört ein Verfahren zur Herstellung eines Trockenextraktes aus Früchten von Piper cubeba L. als wirksamer Bestandteil in einem Medikament, wobei man
- in einem ersten Schritt die Früchte von Piper cubeba L. zur Entfernung der ätherischen Öle entweder
- einer Wasserdampfdestillation unterzieht und das Destillat entfernt oder
- wenigstens einmal mit einer lipophilen Phase extrahiert und diesen lipophilen Extrakt oder diese lipophilen Extrakte entfernt,
- in einem zweiten Schritt die so behandelten Früchte wenigstens einmal entweder mit wenigstens einem Alkohol oder mit einem Gemisch aus wenigstens einem Alkohol und Wasser extrahiert, und
- in einem dritten Schritt die extrahierten Fruchtteile entfernt, den so erhaltenen Extrakt nach der Zugabe eines Hilfsstoffes zuerst auf eine Konzentration an Alkohol zwischen 0,1 und 10 m/m % zu einem Spissumextrakt konzentriert, dann trocknet und gewinnt.

Die Erfindung umfasst auch eine Verwendung eines Extraktes, welcher gemäss dem in diesem Dokument beschriebenen Verfahren erhältlich ist, als wirksamer Bestandteil zur Herstellung eines Medikamentes zur Behandlung wenigstens einer Krankheit, ausgewählt aus der Gruppe bestehend aus Krebserkrankungen, insbesondere Prostatakrebs, Hodenkrebs, Brustkrebs, Gebärmutterkrebs, einschliesslich deren Metastasen, und benigner Prostatahyperplasie.

Darüber hinaus hat die Erfindung die Verwendung eines Extraktes zum Gegenstand, welcher gemäss dem in diesem Dokument beschriebenen Verfahren erhältlich ist, wobei dieser Extrakt die Wirkungen des Sexualhormons Dihydrotestosteron, abgekürzt mit DHT, antagonisiert (insbesondere dessen proliferationssteigernde und anti-apoptotische Wirkung auf Prostatakrebszellen), zur Herstellung eines Medikamentes zur Behandlung von Prostatakrebs, einschliesslich dessen Metastasen, oder von benigner Prostatahyperplasie.

Schliesslich betrifft die Erfindung auch ein Medikament zur Verwendung bei der Behandlung wenigstens einer Krankheit, ausgewählt aus der Gruppe bestehend aus: Krebserkrankungen, insbesondere Prostatakrebs, Hodenkrebs, Brustkrebs, Gebärmutterkrebs, einschliesslich deren Metastasen, und benigner Prostatahyperplasie, wobei es einen Extrakt aus Piper cubeba L. als wirksame Bestandteile enthält und dieser Extrakt anti-androgene und/oder anti-östrogene Wirkungen aufweist. Die wirksamen Bestandteile sind dabei in einem Extrakt enthalten, welcher gemäss dem in diesem Dokument beschriebenen Verfahren erhältlich ist.

Bevorzugte Ausführungsformen werden in den abhängigen Ansprüchen definiert.

Im folgenden Teil werden mögliche Ausführungsformen der vorliegenden Erfindung beschrieben.

Dabei wird auch Bezug auf die Figuren genommen.
Figur 1a zeigt den anti-proliferativen Effekt des gemäss Beispiel 1 hergestellten Extraktes auf LNCap und PC-3 Zellen.
Figur 1b zeigt den anti-proliferativen Effekt der Reinsubstanz Cubebin auf LNCap und PC-3 Zellen.
Figur 2 zeigt die Hemmung der DNA-Synthese von LNCap Zellen durch den gemäss Beispiel 1 hergestellten Extrakt.
Figur 3 zeigt den anti-androgenen Effekt des gemäss Beispiel 1 hergestellten Extraktes auf die androgen-abhängige Zellproliferation auf LNCap Zellen.
Figur 4 zeigt den anti-androgenen Effekt des gemäss Beispiel 1 hergestellten Extraktes auf die DNA-Synthese von LNCap Zellen.
Figur 5 zeigt den anti-östrogenen Effekt des gemäss Beispiel 1 hergestellten Extraktes auf die DNA-Synthese von MCF-7 Zellen.
Figur 6a zeigt den hemmenden Effekt des gemäss Beispiel 1 hergestellten Extraktes und der Reinsubstanz Cubebin auf die Aktivität der 5α-Reduktase Typ II.
Figur 6b zeigt den hemmenden Effekt des bekannten 5α-Reduktase-Inhibitors "Finasterid" auf die Aktivität der 5α-Reduktase Typ II.
Figur 7a zeigt, dass TNF-α die Apoptose der Tumorzellen in Abhängigkeit von der Dosis induziert, und dass dieser Effekt durch DHT bei den Tumorzellen komplett aufgehoben wird.
Figur 7b zeigt, dass die anti-apoptotische Wirkung von DHT durch den gemäss Beispiel 1 hergestellten Extrakt aufgehoben wird.
Figur 8 zeigt, dass sowohl der gemäss Beispiel 1 hergestellte Extrakt als auch die Reinsubstanz Cubebin die Sekretion des Prostata-spezifischen Antigens (PSA) in Abhängigkeit von der jeweiligen Dosis hemmen.
Figur 9 zeigt, dass der gemäss Beispiel 1 hergestellte Extrakt die durch DHT induzierte Sekretion des Prostata-spezifischen Antigens (PSA) stark inhibiert.
Figur 10 zeigt, dass die Androgenrezeptor-Dichte in LNCap Zellen sowohl durch die Behandlung mit dem gemäss Beispiel 1 hergestellten Extrakt als auch durch die Behandlung mit der Reinsubstanz Cubebin dosisabhängig zunehmend reduziert wird.

Die nachfolgenden Beispiele illustrieren die vorliegende Erfindung.

### Beispiel 1 (Herstellung eines FlüssigExtraktes)

110 g unreife, getrocknete Früchte von Piper cubeba L. mit einer Mahlfeinheit von 0,1 mm bis 0,9 mm wurden bei einer Temperatur zwischen 10°C und 20°C während 8 Stunden mit 0,5 Litern Hexan unter Rühren extrahiert. Die mit den aetherischen Ölen und hochlipophilen Stoffen beladene Hexanphase wurde anschliessend abgetrennt. Dieser Vorgang wurde nochmals durchgeführt, wobei die Extraktionszeit auf 2 Stunden begrenzt wurde.

Die so entölten Früchte wurden nun im Vakuumschrank bei einer Temperatur von 40°C bis zur Gewichtskonstanz getrocknet. Man erhielt 92 g entöltes Drogenmaterial.

Anschliessend wurden die so behandelten Früchte bei einer Temperatur zwischen 20°C und 30°C während 2 Stunden mit einem Gemisch aus 90 Gewichtsteilen Ethanol und 10 Gewichtsteilen Wasser unter Rühren extrahiert.

Das Gewichtsverhältnis von Droge zu Extraktionsmittelgemisch betrug 1:5.

Die so extrahierte Droge wurde mittels Schichtenfiltration abgetrennt. Man erhielt 380 g dunkelbraunen Flüssigextrakt mit einem Trockensubstanzgehalt von 1,92 m/m %, entsprechend einer Extraktivstoffausbeute von 7,3 g absolut aus 92 g entölten Früchten.

Dieser Extrakt wird im folgenden Teil mit P9605 bezeichnet.

Dieser Extrakt enthält 20 m/m % an Cubebin, bezogen auf den Trockensubstanzgehalt.

### Beispiel 2 (Herstellung eines Trockenextraktes)

Ein gemäss Beispiel 1 gewonnener Flüssigextrakt wurde in einen Verdampfer bei einer Temperatur von 40°C zudosiert, und die Einengung wurde unter Vakuum (300 mbar bis 20 mbar) und erhöhter Temperatur (40°C bis 55°C) gestartet.

Während der Destillation wurde der verbleibende Teil des Fluidextraktes kontinuierlich in den Verdampfer so lange zudosiert, bis die gesamte Menge des Fluidextraktes eingezogen und bis im erhaltenen Spissumextrakt ein Trockensubstanzgehalt von 30 bis 40 m/m % erreicht war.

Man erhielt 20,0 g Spissumextrakt von dunkelbrauner Farbe, freifliessend und von homogener Konsistenz. Der Spissumextrakt wies einen Trockensubstanzgehalt von 36,5 m/m % auf, was einem Extraktivstoffgehalt von 7,3 g entspricht.

Dieser konzentrierte Spissumextrakt wurde mit 7,8 g einer wässrigen 40 m/m % Gummi arabicum Lösung homogen vermischt und anschliessend in einem Trockner unter Vakuum bei einem Druck von 150 mbar bis 10 mbar und einer Temperatur von 40°C bis 55°C getrocknet.

Man erhielt 10,4 g an ockerbraunem Trockenextrakt mit einem Gehalt an 30 m/m % an Gummi arabicum als Hilfsstoff.

### Beispiel 3 (Herstellung einer Trockenextrakt-Ölsuspension)

Ein gemäss Beispiel 1 gewonnener Flüssigextrakt wurde in einen Verdampfer bei einer Temperatur von 40°C zudosiert, und die Einengung wurde unter Vakuum (300 mbar bis 20 mbar) und erhöhter Temperatur (40°C bis 55°C) gestartet.

Während der Destillation wurde der verbleibende Teil des Fluidextraktes kontinuierlich in den Verdampfer so lange zudosiert, bis die gesamte Menge des Fluidextraktes eingezogen und bis im erhaltenen Spissumextrakt ein Trockensubstanzgehalt von 10 bis 20 m/m % erreicht war.

Man erhielt 54,0 g Spissumextrakt von dunkelbrauner Farbe, freifliessend und von homogener Konsistenz. Der Spissumextrakt wies einen Trockensubstanzgehalt von 15,7 m/m % auf, was einem Extraktivstoffgehalt von 7,3 g entspricht.

Dieser dünnflüssige Spissumextrakt wurde mit 6,8 g Mittelkettiger Triglyceride (Ph. Eur.) und 0,5 g Soja-Lecithin (ÖAB 90) vermischt und in einen Verdampfer bei einer Temperatur von 40°C zudosiert. Die Einengung dieses Gemisches wurde unter Vakuum (300 mbar bis 40 mbar) und erhöhter Temperatur (40°C bis 50°C) so lange durchgeführt, bis im erhaltenen Spissumextrakt ein Trockensubstanzgehalt von 70 bis 80 m/m % erreicht war.

Man erhielt einen dickflüssigen Spissumextrakt, der anschliessend in einem Trockner unter Vakuum bei einem Druck von 150 mbar bis 10 mbar und einer Temperatur von 40°C bis 55°C getrocknet wurde, bis ein Trockensubstanzgehalt von 99,5 m/m % erreicht war.

Man erhielt 14,9 g an dunkelbrauner Trockenextrakt-Ölsuspension mit einem Gehalt an 49 m/m % an Mittelkettigen Triglyceriden und 3,36 m/m % Soja-Lecithin als Hilfsstoffe.

### Beispiel 4 (Hemmung der Zellproliferation)

Mit dem gemäss Beispiel 1 hergestellten Flüssigextrakt P9605 wurden Zellproliferationstests durchgeführt. Als Kontrolle wurde ein typischer Inhaltsstoff der Kubeben-Früchte, das Lignan Cubebin, mitgeführt.

Zur Messung der Hemmung der Zellproliferation wurde dieser Extrakt zu LNCap und zu PC-3 Zellen zugegeben. Die so behandelten Zellen wurden während 4 Tagen in 10% FBS Kulturmedium kultiviert.

Zum Vergleich wurde das reine Lignan Cubebin, das im erfindungsgemäss hergestellten Extrakt gemäss Beispiel 1 zu 20 m/m % der Trockenmasse enthalten ist, ebenfalls zu LNCap und zu PC-3 Zellen zugegeben. Die so behandelten Zellen wurden während 4 Tagen in 10% FBS Kulturmedium kultiviert.

Dabei wurde gemäss T. Lindl, Zell- und Gewebekultur, 4. überarbeitete Auflage, 2000, Spektrum Akademischer Verlag, Heidelberg vorgegangen.

Alle erhaltenen Daten sind in Prozent zur Lösungsmittelkontrolle (Test ohne Extrakt und ohne Cubebin) angegeben; es sind die Mittelwerte mit Standardabweichungen aus 4 Experimenten mit 3-facher Wiederholung angegeben.

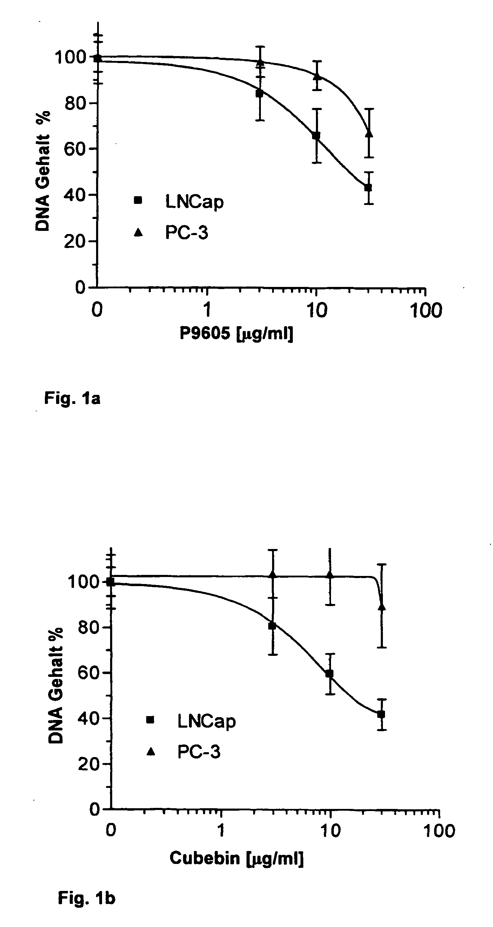

Aus den in den Figuren 1a und 1b gezeigten Daten ist ersichtlich, dass sowohl der erfindungsgemäss hergestellte Extrakt als auch die Reinsubstanz Cubebin einen anti-proliferativen Effekt sowohl bei LNCap als auch PC-3 Zellen in Abhängigkeit von der jeweiligen Dosis zeigen.

Die Hemmung ist bei den LNCap-Zellen stärker ausgeprägt als bei den PC-3 Zellen.

Wie aus den Figuren 1a und 1b ersichtlich ist, ist die Hemmwirkung des gemäss Beispiel 1 hergestellten Extraktes P9605 um ein Mehrfaches stärker als dies durch seinen Gehalt an Cubebin erklärbar wäre. Der Extrakt enthält lediglich 20 m/m % an Cubebin, weist aber eine gleichstarke (LNCap) oder stärkere Hemmwirkung (PC-3) auf.

### Beispiel 5 (Hemmung der DNA Synthese)

Mit dem gemäss Beispiel 1 hergestellten Flüssigextrakt P9605 wurden DNA-Synthesetests durchgeführt.

Zur Messung der Hemmung der DNA Synthese wurde der erfindungsgemäss hergestellte Extrakt zu LNCap Zellen zugegeben. Die so behandelten Zellen wurden während 4 Tagen in 10% FBS Kulturmedium kultiviert.

Anschliessend wurde die Menge an eingebautem ³H-Thymidin gemessen.

Dabei wurde gemäss T. Lindl, Zell- und Gewebekultur, 4. überarbeitete Auflage, 2000, Spektrum Akademischer Verlag, Heidelberg vorgegangen.

Alle erhaltenen Daten sind in Prozent zur Lösungsmittelkontrolle (Test ohne Extrakt) angegeben; es sind die Mittelwerte mit Standardabweichungen aus 4 Experimenten mit 3-facher Wiederholung angegeben.

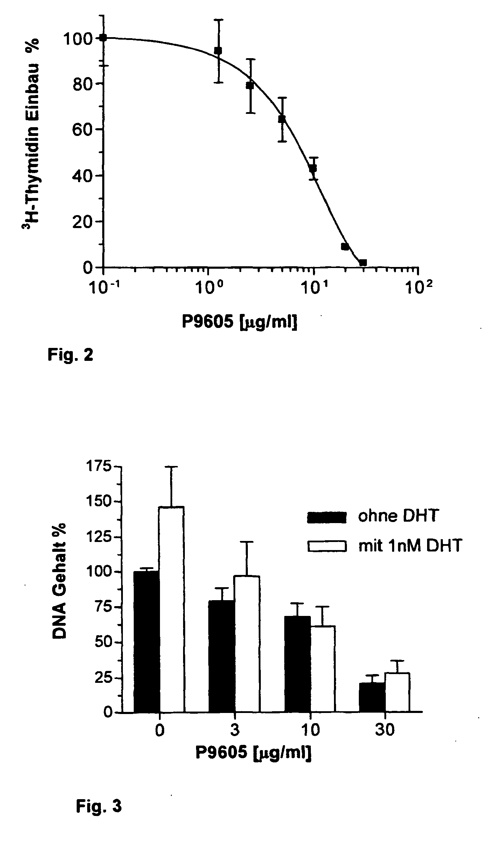

Aus den in Figur 2 gezeigten Daten ist ersichtlich, dass der erfindungsgemäss hergestellte Extrakt die DNA-Synthese in Abhängigkeit der jeweiligen Dosis hemmt.

### Beispiel 6 (Anti-androgener Effekt auf die Zellproliferation)

Der anti-androgene Effekt auf die androgenabhängige Zellproliferation des gemäss Beispiel 1 hergestellten Flüssigextraktes P9605 wurde bestimmt.

Dabei wurde der erfindungsgemäss hergestellte Extrakt zu LNCap Zellen zugegeben. Die so behandelten Zellen wurden während 6 Tagen in 10% CSS Kulturmedium kultiviert.

Diese Kultivierung erfolgte einmal ohne die Hinzugabe von Dihydrotestosteron, abgekürzt mit DHT, und einmal mit der Hinzugabe von 1 nM DHT.

Anschliessend wurde der Einfluss des erfindungsgemäss hergestellten Extraktes auf die Zellproliferation der Tumorzellen anhand des DNA-Gehaltes bestimmt.

Alle erhaltenen Daten sind in Prozent zur Lösungsmittelkontrolle (Test ohne Extrakt) angegeben; es sind die Mittelwerte mit Standardabweichungen aus 4 Experimenten mit 3-facher Wiederholung angegeben.

Aus den in Figur 3 gezeigten Daten ist ersichtlich, dass der erfindungsgemäss hergestellte Extrakt den stimulierenden Effekt von DHT auf die Zellproliferation der Tumorzellen dosisabhängig übersteuert und darüber hinaus die basale Proliferation der Zellen absenkt.

Es ist bekannt, dass DHT die Zellproliferation steigert; siehe den Kontrollwert bei null.

### Beispiel 7 (Anti-androgener Effekt auf die DNA-Synthese)

Der anti-androgene Effekt auf die DNA-Synthese des gemäss Beispiel 1 hergestellten Flüssigextraktes P9605 wurde bestimmt.

Dabei wurde der erfindungsgemäss hergestellte Extrakt zu LNCap Zellen zugegeben. Die so behandelten Zellen wurden während 6 Tagen in 10% CSS Kulturmedium kultiviert.

Diese Kultivierung erfolgte einmal ohne die Hinzugabe von Dihydrotestosteron, abgekürzt mit DHT, und einmal mit der Hinzugabe von 1 nM DHT.

Anschliessend wurde die Menge an eingebautem ³H-Thymidin gemessen.

Dabei wurde gemäss T. Lindl, Zell- und Gewebekultur, 4. überarbeitete Auflage, 2000, Spektrum Akademischer Verlag, Heidelberg vorgegangen.

Alle erhaltenen Daten sind in Prozent zur Lösungsmittelkontrolle (Test ohne Extrakt) angegeben; es sind die Mittelwerte mit Standardabweichungen aus 4 Experimenten mit 3-facher Wiederholung angegeben.

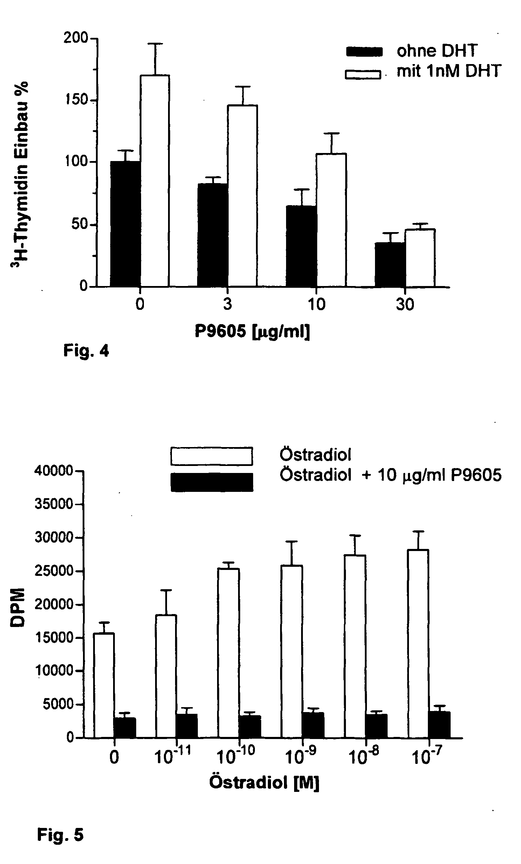

Aus den in Figur 4 gezeigten Daten ist ersichtlich, dass der erfindungsgemäss hergestellte Extrakt den stimulierenden Effekt von DHT auf die DNA-Synthese der Tumorzellen dosisabhängig übersteuert und darüber hinaus die basale DNA-Synthese der Zellen absenkt.

Es ist bekannt, dass DHT die DNA-Synthese steigert; siehe den Kontrollwert bei null.

### Beispiel 8 (Anti-östrogener Effekt auf die DNA-Synthese von Brustkrebszellen)

Der anti-östrogene Effekt auf die DNA-Synthese von Brustkrebszellen des gemäss Beispiel 1 hergestellten Flüssigextraktes P9605 wurde bestimmt.

Dabei wurden MCF-7 Zellen während 3 Tagen in 10% CSS Kulturmedium, welchem unterschiedliche Konzentrationen von Östradiol zugegeben wurden, kultiviert.

Diese Kultivierung erfolgte einmal ohne die Hinzugabe von erfindungsgemäss hergestelltem Extrakt und einmal mit der Hinzugabe von 10 µg/ml an Extrakt.

Anschliessend wurde die Menge an eingebautem ³H-Thymidin gemessen.

Dabei wurde gemäss T. Lindl, Zell- und Gewebekultur, 4. überarbeitete Auflage, 2000, Spektrum Akademischer Verlag, Heidelberg vorgegangen.

Alle erhaltenen Daten sind in DPM (radioaktiver Zerfall pro Minute; degradation per minute) angegeben; es sind die Mittelwerte mit Standardabweichungen aus 4 Experimenten mit 3-facher Wiederholung angegeben.

Aus den in Figur 5 gezeigten Daten ist ersichtlich, dass der erfindungsgemäss hergestellte Extrakt die Stimulation der DNA-Synthese von Brustkrebszellen durch Östradiol komplett oder nahezu komplett unterbindet.

Es ist bekannt, dass Östradiol die DNA-Synthese von Brustkrebszellen steigert; siehe den Kontrollwert bei null.

### Beispiel 9 (Inhibition der 5α-Reduktase Typ II Aktivität)

Die Inhibition der 5α-Reduktase Typ II Aktivität mittels des gemäss Beispiel 1 hergestellten Flüssigextraktes P9605 wurde bestimmt.

Der Assay wurde mit einem Homogenat aus HEK293 Zellen durchgeführt, welche die 5α-Reduktase Typ II überexprimieren (Reichert W., Hartmann R.W. und Jose J.; 2001, Journal Enzyme Inhibition, Vol. 16, 47-53).

Der Einfluss des erfindungsgemäss hergestellten Extraktes und der Reinsubstanz Cubebin auf die Aktivität der 5α-Reduktase Typ II wurde mittels der Bestimmung der Umwandlung von ³H-Testosteron zu ³H-DHT bestimmt.

Als Kontrollsubstanz diente der bekannte 5α-Reduktase-Inhibitor "Finasterid".

Alle erhaltenen Daten sind in Prozent zur Lösungsmittelkontrolle (Test ohne Extrakt) angegeben; es sind die Mittelwerte mit Standardabweichungen aus 4 Experimenten mit 3-facher Wiederholung angegeben.

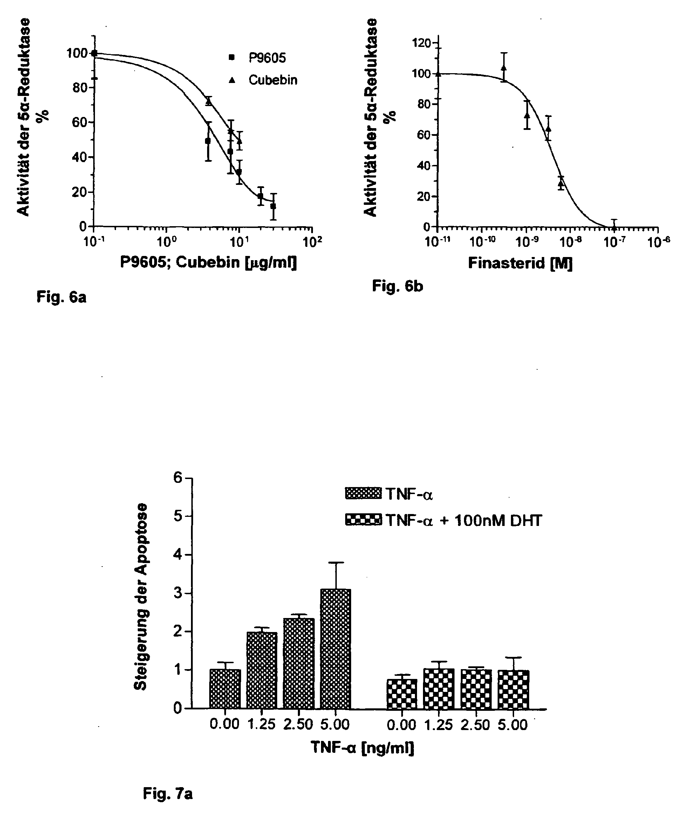

Aus den in Figur 6a gezeigten Daten ist ersichtlich, dass sowohl der erfindungsgemäss hergestellte Extrakt als auch die Reinsubstanz Cubebin einen hemmenden Effekt auf die Aktivität der 5α-Reduktase Typ II zeigen.

Die Hemmung ist beim Extrakt stärker als bei der Reinsubstanz Cubebin.

Der Extrakt hemmt mit einem IC₅₀-Wert von 3,6 µg/ml, währenddem die Reinsubstanz Cubebin mit einem IC₅₀-Wert von 9, 9 µg/ml hemmt.

Der Verlauf der Dosis-Wirkungskurve des Extraktes und der Reinsubstanz Cubebin sind analog zum Verlauf der Dosis-Wirkungskurve des bekannten 5α-Reduktase-Inhibitors "Finasterid" (Figur 6b).

### Beispiel 10 (Steigerung von Apoptose)

Die Induktion von Apoptose mittels des gemäss Beispiel 1 hergestellten Flüssigextraktes P9605 wurde bestimmt.

Als Vorversuch wurde zur Messung der Induktion der Apoptose der Tumor-Nekrose-Faktor TNF-α alleine sowie in Kombination mit 100 nM Dihydrotestosteron, abgekürzt mit DHT, zu LNCap Zellen zugegeben. Die so behandelten Zellen wurden während 2 Tagen in 10% FBS Kulturmedium kultiviert.

Die Apoptose der Zellen wurde mittels Anwendung eines kommerziellen Apoptose-Immuno-Assay-Kits gemessen, bei dem spezifisch die DNA- und Histon-Fragmente, die als Mono- und Oligonucleosome vorliegen, detektiert werden.

Aus den in Figur 7a gezeigten Daten ist ersichtlich, dass TNF-α die Apoptose der Tumorzellen in Abhängigkeit von der Dosis induziert.

Dieser Effekt wird durch DHT bei den Tumorzellen komplett oder nahezu komplett aufgehoben.

Analoge Versuche wurden mit DHT alleine sowie in Kombination mit 10 µg/ml des erfindungsgemäss hergestellten Extraktes durchgeführt.

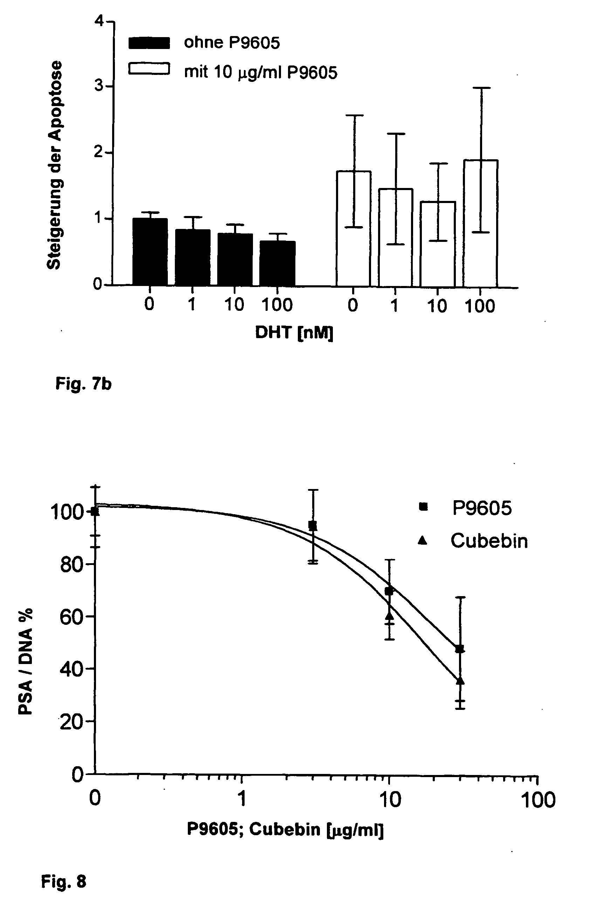

Aus den in Figur 7b gezeigten Daten ist ersichtlich, dass die anti-apoptotische Wirkung von DHT durch den erfindungsgemäss hergestellten Extrakt aufgehoben wird.

### Beispiel 11 (Inhibition der Sekretion des Prostata-spezifischen Antigens)

Die Inhibition des Prostata-spezifischen Antigens (PSA) mittels des gemäss Beispiel 1 hergestellten Flüssigextraktes P9605 wurde bestimmt.

Dabei wurden in einem Versuch LNCap Zellen während 2 Tagen in 10% CSS Kulturmedium, welchem unterschiedliche Konzentrationen entweder des erfindungsgemäss hergestellten Extraktes oder der Reinsubstanz Cubebin zugegeben wurden, kultiviert.

Anschliessend wurde die sekretierte PSA Menge im Zellüberstand mittels eines Immuno-Assays gemessen. Zusätzlich wurde die DNA-Menge bestimmt.

In Figur 8 wird das Verhältnis der Menge PSA zur Menge DNA in Prozent angegeben.

Es sind die Mittelwerte mit Standardabweichungen aus 4 Experimenten mit 3-facher Wiederholung angegeben.

Aus den in Figur 8 gezeigten Daten ist ersichtlich, dass sowohl der Extrakt als auch die Reinsubstanz Cubebin die Sekretion des Prostata-spezifischen Antigens (PSA) in Abhängigkeit von der jeweiligen Dosis hemmen.

In einem zweiten Versuch wurden LNCap Zellen während 2 Tagen in 10% CSS Kulturmedium, welchem unterschiedliche Konzentrationen von Dihydrotestosteron, abgekürzt mit DHT, zugegeben wurden, kultiviert.

Diese Kultivierung erfolgte einmal ohne die Hinzugabe von erfindungsgemäss hergestelltem Extrakt und einmal mit der Hinzugabe von 10 µg/ml an Extrakt.

Anschliessend wurde die sekretierte PSA Menge im Zellüberstand mittels eines Immuno-Assays gemessen. Zusätzlich wurde die DNA-Menge bestimmt.

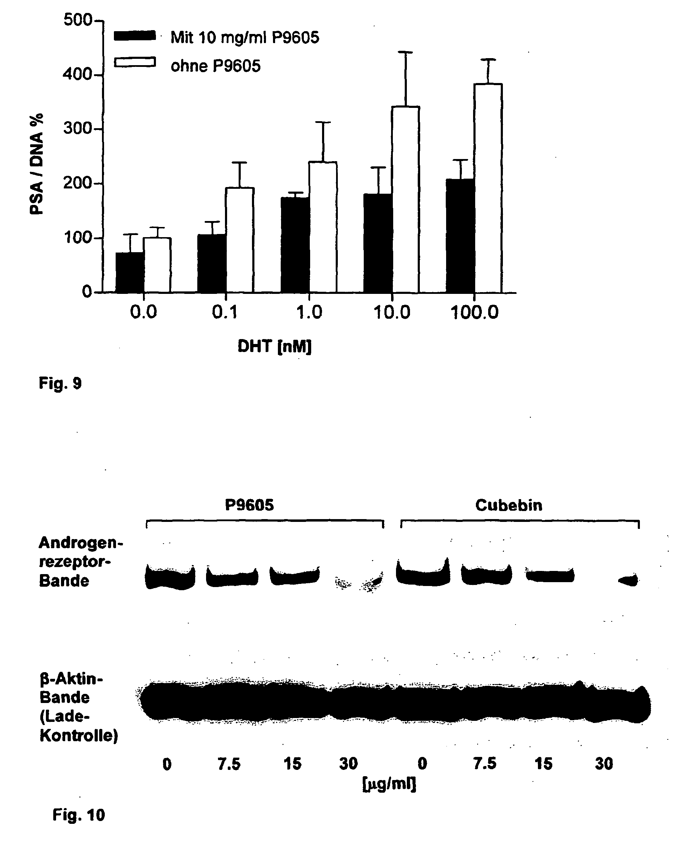

In Figur 9 wird das Verhältnis der Menge PSA zur Menge DNA in Prozent angegeben.

Es sind die Mittelwerte mit Standardabweichungen aus 4 Experimenten mit 3-facher Wiederholung angegeben.

Aus den in Figur 9 gezeigten Daten ist ersichtlich, dass die durch DHT induzierte Sekretion des Prostata-spezifischen Antigens (PSA) durch den erfindungsgemäss hergestellten Extrakt stark inhibiert wird.

### Beispiel 12 (Bildung von Androgenrezeptoren)

Die Beeinflussung der Bildung von Antrogenrezeptoren mittels des gemäss Beispiel 1 hergestellten Flüssigextraktes P9605 wurde bestimmt.

Dabei wurden in einem Versuch LNCap Zellen während 2 Tagen in 10% FBS Kulturmedium, welchem unterschiedliche Konzentrationen entweder des erfindungsgemäss hergestellten Extraktes oder der Reinsubstanz Cubebin zugegeben wurden, kultiviert.

Anschliessend wurde die Änderung der Androgenrezeptor-Menge mittels Westernblot Analyse bestimmt.

In Figur 10 sind die Banden des Androgenrezeptors gezeigt.

Die Androgenrezeptor-Dichte in LNCap Zellen wird sowohl durch die Behandlung mit dem erfindungsgemäss hergestellten Extrakt als auch durch die Behandlung mit der Reinsubstanz Cubebin dosisabhängig zunehmend reduziert.

### Schlussfolgerungen

Anhand der Beispiele 1 bis 3 wird aufgezeigt, durch welche Kombinationen von Verfahrensschritten sich Extrakte aus Kubeben-Früchten herstellen lassen, die frei oder nahezu frei von aetherischem Öl sind, neue Eigenschaften aufweisen und die Ziele der vorliegenden Erfindung erreichen.

Die Beispiele 4 bis 12 demonstrieren die Antitumoraktivität der erfindungsgemäss hergestellten Extrakte und illustrieren die Wirkmechanismen, die der Aktivität gegenüber hormonabhängigen Tumorzellen zugrunde liegen. Diese Beispiele zeigen das hohe therapeutische Potenzial der erfindungsgemäss hergestellten Extrakte, insbesondere zur Therapie von malignen Erkrankungen, deren Progression von weiblichen oder männlichen Sexualhormonen beeinflusst wird.

Betrachtet man die Wirkstärke (IC₅₀: 3,6 µg/ml) der erfindungsgemäss hergestellten Extrakte gegenüber der humanen 5α-Reduktase im Vergleich zu der in JP 2000-095649 A (IC₅₀:790 µg/ml) angeführten Aktivität, so zeigt sich, dass die erfindungsgemäss hergestellten Extrakte eine etwa 200-fach höhere Aktivität aufweisen und somit auch für die Behandlung der Prostatahyperplasie vollkommen neue Möglichkeiten eröffnen.

## Patentansprüche

1. Verfahren zur Herstellung eines Trockenextraktes aus Früchten von Piper cubeba L. als wirksamer Bestandteil in einem Medikament,
**dadurch gekennzeichnet, dass** man
- in einem ersten Schritt die Früchte von Piper cubeba L. zur Entfernung der ätherischen Öle entweder
- - einer Wasserdampfdestillation unterzieht und das Destillat entfernt oder
- - wenigstens einmal mit einer lipophilen Phase extrahiert und diesen lipophilen Extrakt oder diese lipophilen Extrakte entfernt,
- in einem zweiten Schritt die so behandelten Früchte wenigstens einmal entweder mit wenigstens einem Alkohol oder mit einem Gemisch aus wenigstens einem Alkohol und Wasser extrahiert, und
- in einem dritten Schritt die extrahierten Fruchtteile entfernt, den so erhaltenen Extrakt nach der Zugabe eines Hilfsstoffes zuerst auf eine Konzentration an Alkohol zwischen 0,1 und 10 m/m % zu einem Spissumextrakt konzentriert, dann trocknet und gewinnt.

2. Verfahren nach Anspruch 1, **dadurch gekennzeichnet, dass** die unreifen Früchte von Piper cubeba L. verwendet werden, und unmittelbar vor der Extraktion gemahlen und in gemahlener Form extrahiert werden, insbesondere mit einer Mahlfeinheit von 0,1 mm bis 0,9 mm.

3. Verfahren nach einem der Ansprüche 1 bis 2, **dadurch gekennzeichnet, dass** im ersten Schritt als lipophile Phase entweder überkritisches CO₂ oder ein gerader oder verzweigter Kohlenwasserstoff mit 4 bis 9 Kohlenstoffatomen, insbesondere Hexan oder Isopentan, verwendet wird.

4. Verfahren nach einem der Ansprüche 1 bis 3, **dadurch gekennzeichnet, dass** im ersten Schritt pro Gewichtsteil zu extrahierende Früchte von 1 bis 20 Gewichtsteile, insbesondere von 6 bis 12 Gewichtsteile, an lipophiler Phase eingesetzt werden.

5. Verfahren nach einem der Ansprüche 1 bis 4, **dadurch gekennzeichnet, dass** im ersten Schritt die Extraktion mit der lipophilen Phase bei einer Temperatur von 0°C bis 50°C, insbesondere von 5°C bis 15°C, und während einer Zeit von 2 bis 4 Stunden erfolgt.

6. Verfahren nach einem der Ansprüche 1 bis 5, **dadurch gekennzeichnet, dass** im zweiten Schritt der Alkohol ein Alkohol mit 1 bis 5 Kohlenstoffatomen ist, insbesondere Ethanol, und dass das Gemisch aus wenigstens einem Alkohol und Wasser aus 50 bis 90 m/m % Alkohol und 50 bis 10 m/m % Wasser, vorzugsweise aus 80 bis 90 m/m % Alkohol und 20 bis 10 m/m % Wasser, besteht, wobei Ethanol bevorzugt ist.

7. Verfahren nach einem der Ansprüche 1 bis 6, **dadurch gekennzeichnet, dass** im zweiten Schritt pro Gewichtsteil zu extrahierende Früchte von 1 bis 20 Gewichtsteile, insbesondere von 6 bis 12 Gewichtsteile, an wenigstens einem Alkohol oder einem Gemisch aus wenigstens einem Alkohol und Wasser eingesetzt werden.

8. Verfahren nach einem der Ansprüche 1 bis 7, **dadurch gekennzeichnet, dass** im zweiten Schritt die Extraktion mit wenigstens einem Alkohol oder mit einem Gemisch aus wenigstens einem Alkohol und Wasser bei einer Temperatur von 20°C bis 60°C und während einer Zeit von 2 bis 4 Stunden erfolgt.

9. Verfahren nach einem der Ansprüche 1 bis 8, **dadurch gekennzeichnet, dass** der im dritten Schritt verwendete Hilfsstoffes ein Tröcknungshilfsstoff ist, beispielsweise Mannitol, und dass auf eine Konzentration an Alkohol von 5 m/m % konzentriert wird, und dass die Trocknung durch Sprühtrocknung, Bandtrocknung oder Schaufeltrocknung erfolgt.

10. Verfahren nach einem der Ansprüche 1 bis 9, **dadurch gekennzeichnet, dass** der im dritten Schritt erhaltene Extrakt frei oder nahezu frei von α-Cubeben (α-cubebene) und β-Cubeben (β-cubebene) ist.

11. Verwendung eines Extraktes, welcher gemäss dem Verfahren nach einem der Ansprüche 1 bis 10 erhältlich ist, als wirksamer Bestandteil zur Herstellung eines Medikamentes zur Behandlung wenigstens einer Krankheit, ausgewählt aus der Gruppe, bestehend aus Krebserkrankungen, insbesondere Prostatakrebs, Hodenkrebs, Brustkrebs, Gebärmutterkrebs, einschliesslich deren Metastasen, und benigner Prostatahyperplasie.

12. Verwendung eines Extraktes, welcher gemäss dem Verfahren nach einem der Ansprüche 1 bis 10 erhältlich ist, wobei dieser Extrakt die Wirkungen des Sexualhormons Dihydrotestosteron, abgekürzt mit DHT, antagonisiert, insbesondere dessen proliferationssteigernde und anti-apoptotische Wirkung auf Prostatakrebszellen, als wirksamer Bestandteil zur Herstellung eines Medikamentes zur Behandlung von Prostatakrebs, einschliesslich dessen Metastasen, oder von benigner Prostatahyperplasie.

13. Medikament zur Verwendung bei der Behandlung wenigstens einer Krankheit, ausgewählt aus der Gruppe, bestehend aus Krebserkrankungen, insbesondere Prostatakrebs, Hodenkrebs, Brustkrebs, Gebärmutterkrebs, einschliesslich deren Metastasen, und benigner Prostatahyperplasie, **dadurch gekennzeichnet, dass** es einen Extrakt aus Piper cubeba L. als wirksame Bestandteile enthält, wobei dieser Extrakt anti-androgene und/oder anti-östrogene Wirkungen aufweist,
und wobei,
die wirksamen Bestandteile in einem Extrakt enthalten sind, welcher gemäss dem Verfahren nach einem der Ansprüche 1 bis 10 erhältlich ist.

14. Verwendung eines Extraktes, welcher gemäss dem Verfahren nach einem der Ansprüche 1 bis 10 erhältlich ist, wobei dieser Extrakt die Wirkungen des Sexualhormons Dihydrotestosteron, abgekürzt mit DHT, antagonisiert, insbesondere dessen proliferationssteigernde und anti-apoptotische Wirkung auf Prostatakrebszellen, zur Herstellung eines Medikamentes zur Behandlung von Prostatakrebs, einschliesslich dessen Metastasen, oder von benigner Prostatahyperplasie.

## Claims

1. A process for the preparation of a dry extract of fruits of Piper cubeba L. as active component in a medicament,
**characterized in that**
- in a first step for the removal of the essential oils the fruits of Piper cubeba L. either
- - are subjected to a steam distillation and the distillate is removed, or
- - are extracted at least once with a lipophilic phase and this lipophilic extract or these lipophilic extracts is (are) removed,
- in a second step the so treated fruits are extracted at least once either with at least one alcohol or with a mixture of at least one alcohol and water, and
- in a third step the extracted fruit parts are removed, the so obtained extract after the addition of an auxiliary agent is first concentrated to a concentration of alcohol between 0.1 and 10 m/m % to a spissum extract, is then dried and obtained.

2. The process according to claim 1, **characterized in that** the immature fruits of Piper cubeba L. are used, and are grinded immediately before the extraction and are extracted in grinded form, especially with a grinding fineness from 0.1 mm to 0.9 mm.

3. The process according to one of claims 1 to 2, **characterized in that** in the first step as lipophilic phase is used either supercritical CO₂ or a straight or branched hydrocarbon having 4 to 9 carbon atoms, especially hexane or isopentane.

4. The process according to one of claims 1 to 3, **characterized in that** in the first step per part by weight of fruits to be extracted are used from 1 to 20 parts by weight, especially from 6 to 12 parts by weight, of lipophilic phase.

5. The process according to one of claims 1 to 4, **characterized in that** in the first step the extraction with the lipophilic phase is realized at a temperature from 0°C to 50°C, especially from 5°C to 15°C, and during a time from 2 to 4 hours.

6. The process according to one of claims 1 to 5, **characterized in that** in the second step the alcohol is an alcohol having 1 to 5 carbon atoms, especially ethanol, and that the mixture of at least one alcohol and water consists of 50 to 90 m/m % of alcohol and 50 to 10 m/m % of water, preferably of 80 to 90 m/m % of alcohol and 20 to 10 m/m % of water, whereby ethanol is preferred.

7. The process according to one of claims 1 to 6, **characterized in that** in the second step per part by weight of fruits to be extracted are used from 1 to 20 parts by weight, especially from 6 to 12 parts by weight, of at least one alcohol or of a mixture of at least one alcohol and water.

8. The process according to one of claims 1 to 7, **characterized in that** in the second step the extraction with at least one alcohol or with a mixture of at least one alcohol and water is realized at a temperature from 20°C to 60°C and during a time from 2 to 4 hours.

9. The process according to one of claims 1 to 8, **characterized in that** in the third step the auxiliary agent is a drying auxiliary agent, for example mannitol, and that there is concentrated to a concentration of alcohol of 5 m/m %, and that the drying is a spray drying, a belt drying or a blade drying.

10. The process according to one of claims 1 to 9, **characterized in that** the extract obtained in the third step is free or nearly free of α-cubebene and β-cubebene.

11. Use of an extract that is obtainable according to the process according to one of claims 1 to 10 as active component for the preparation of a medicament for the treatment of at least one disease, selected from the group consisting of cancer diseases, especially prostate cancer, testicles cancer, breast cancer, uterus cancer, including their metastases, and benign prostate hyperplasia.

12. Use of an extract that is obtainable according to the process according to one of claims 1 to 10, whereby this extract antagonize the activities of the sex hormone dihydrotestosterone, abbreviated with DHT, especially its proliferation enhancing and anti-apoptotic activity on prostate cancer cells, as active component for the preparation of a medicament for the treatment of prostate cancer, including its metastases, and of benign prostate hyperplasia.

13. Medicament for the use in the treatment of at least one disease, selected from the group consisting of cancer diseases, especially prostate cancer, testicles cancer, breast cancer, uterus cancer, including their metastases, and of benign prostate hyperplasia, **characterized in that** it contains an extract from Piper cubeba L. as active components, whereby this extract has anti-androgenic and/or anti-estrogenic activities,
and whereby
the active components are contained in an extract that is obtainable according to the process according to one of claims 1 to 10.

14. Use of an extract that is obtainable according to the process according to one of claims 1 to 10, whereby this extract antagonize the activities of the sex hormone dihydrotestosterone, abbreviated with DHT, especially its proliferation enhancing and anti-apoptotic effect on prostate cancer cells, for the preparation of a medicament for the treatment of prostate cancer, including its metastases, or of benign prostate hyperplasia.

## Revendications

1. Procédé pour la fabrication d'un extrait sec à partir de fruits de Piper cubeba L.
comme composant actif dans un médicament,
**caractérisé en ce que**
- dans une première étape, on soumet les fruits de Piper cubeba L., pour éliminer les huiles essentielles
- soit à une distillation à la vapeur d'eau et l'on élimine le distillat,
- soit on extrait au moins une fois avec une phase lipophile et on élimine cet extrait lipophile ou ces extraits lipophiles,
- dans une seconde étape, les fruits ainsi traités sont extraits au moins une fois soit avec au moins un alcool, soit avec un mélange d'au moins un alcool et de l'eau et
- dans une troisième étape, les parties de fruits extraites sont éliminées, l'extrait ainsi obtenu est concentré, après l'addition d'un agent auxiliaire, tout d'abord à une concentration d'alcool entre 0,1 et 10 % m/m, en un extrait mou (Spiss.), est ensuite séché et produit.

2. Procédé selon la revendication 1, **caractérisé en ce que** les fruits non mûrs de Piper cubeba L. sont utilisés et moulus directement avant l'extraction et sont extraits sous forme moulue, en particulier avec un degré de mouture de 0,1 mm à 0,9 mm.

3. Procédé selon l'une des revendications 1 à 2, **caractérisé en ce que**, dans la première étape, on utilise, comme phase lipophile, soit du CO₂ surcritique, soit un hydrocarbure droit ou ramifié avec 4 à 9 atomes de carbone, en particulier de l'hexane ou de l'isopentane.

4. Procédé selon l'une des revendications 1 à 3, **caractérisé en ce que**, dans la première étape, on met en oeuvre, par partie en poids, des fruits à extraire de 1 à 20 parties en poids, en particulier de 6 à 12 parties en poids, dans une phase lipophile.

5. Procédé selon l'une des revendications 1 à 4, **caractérisé en ce que**, dans la première étape, l'extraction se fait avec la phase lipophile à une température de 0 °C à 50 °C, en particulier de 5 °C à 15 °C, et pendant une durée de 2 à 4 heures.

6. Procédé selon l'une des revendications 1 à 5, **caractérisé en ce que**, dans la seconde étape, l'alcool est un alcool avec 1 à 5 atomes de carbone, en particulier de l'éthanol, et que le mélange est constitué par au moins un alcool et de l'eau, par 50 à 90 % m/m d'alcool et 50 à 10 % m/m d'eau, de préférence par 80 à 90 % d'alcool et 20 à 10 % m/m d'eau, l'éthanol étant préféré.

7. Procédé selon l'une des revendications 1 à 6, **caractérisé en ce que**, dans la seconde étape, on met en oeuvre, par partie en poids, des fruits à extraire de 1 à 20 parties en poids, en particulier de 6 à 12 parties en poids, dans au moins un alcool ou un mélange d'au moins un alcool et de l'eau.

8. Procédé selon l'une des revendications 1 à 7, **caractérisé en ce que**, dans la seconde étape, l'extraction se fait avec au moins un alcool ou avec un mélange d'au moins un alcool et de l'eau à une température de 20 °C à 60 °C et pendant une durée de 2 à 4 heures.

9. Procédé selon l'une des revendications 1 à 8, **caractérisé en ce que** l'agent auxiliaire utilisé dans la troisième étape est un auxiliaire de séchage, par exemple du mannitol, et que la concentration se fait à une concentration d'alcool de 5 % m/m et que le séchage se fait par séchage par pulvérisation, par séchage par bande ou par séchage par ailettes.

10. Procédé selon l'une des revendications 1 à 9, **caractérisé en ce que** l'extrait obtenu dans la troisième étape est exempt ou pratiquement exempt d'α-cubébène (α-cubébène) et de β-cubébène (β-cubébène).

11. Utilisation d'un extrait qui peut être obtenu selon le procédé selon l'une des revendications 1 à 10, comme composant actif dans un médicament pour la fabrication d'un médicament pour le traitement d'au moins une maladie sélectionnée dans le groupe comportant les maladies cancéreuses, en particulier le cancer de la prostate, le cancer des testicules, le cancer du sein, le cancer de l'utérus, y compris leurs métastases, et de l'hyperplasie bénigne de la prostate.

12. Utilisation d'un extrait qui peut être obtenu selon le procédé selon l'une des revendications 1 à 10, cet extrait antagonisant les effets de l'hormone sexuelle dihydrotestostérone, en abrégé DHT, en particulier son effet augmentant la prolifération et l'effet anti-apoptotique sur les cellules du cancer de la prostate, comme composant actif pour la fabrication d'un médicament pour le traitement du cancer de la prostate, y compris de ses métastases, ou de l'hyperplasie bénigne de la prostate.

13. Médicament à utiliser pour le traitement d'au moins une maladie sélectionnée dans le groupe comportant les maladies cancéreuses, en particulier le cancer de la prostate, le cancer des testicules, le cancer du sein, le cancer de l'utérus, y compris leurs métastases, et de l'hyperplasie bénigne de la prostate, **caractérisé en ce qu'**il contient un extrait de Piper cubeba L. comme composants actifs, cet extrait présentant des effets anti-androgènes et/ou des effets anti-oestrogènes et les composants actifs étant contenus dans un extrait qui peut êtreobtenu selon le procédé selon l'une des revendications 1 à 10.

14. Utilisation d'un extrait qui peut être obtenu selon le procédé selon l'une des revendications 1 à 10, cet extrait antagonisant les effets de l'hormone sexuelle dihydrotestostérone, en abrégé DHT, en particulier son effet augmentant la prolifération et son effet anti-apoptotique sur les cellules du cancer de la prostate, pour la fabrication d'un médicament pour le traitement du cancer de la prostate, y compris de ses métastases, ou de l'hyperplasie bénigne de la prostate.
